# EUROPEAN PATENT APPLICATION

(11) **EP 3 932 204 A1**
(43) Date of publication of application: **05.01.2022**
(21) Application number: 21182157.4
(22) Date of filing: 28.06.2021
(51) Int. Cl.: A01N 37/02, A01N 37/16, A01N 59/00, A01N 63/14, A01P 1/00

(54) **CHEMICAL STERILIZATION OF HONEY**

(30) Priority: 29.06.2020 US 202063102756 P
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

The present application relates to the process of using parecetic acid and / or performic acid with heating to create a sterilized product. Green tea polyphenols may be used to inhibit spore germination. Honey is one product that may benefit from this process. Other products that may benefit from this process include pickled foods, condiments, salad dressing, marinades, apple products, strawberry products, and raspberry products. Medications containing honey for stomach ulcers may benefit from this sterilization process. Cosmetics containing honey may also benefit from this process.

## Description

### CROSS-REFERENCE TO RELATED APPLICATIONS

This application claims the benefit of U.S. Provisional Application 63/102756, filed on June 29, 2020. The entire disclosure of the above application is incorporated herein by reference.

### FIELD

The present application relates to a sterilized honey and the process of using parecetic acid and / or performic acid with heating to create a sterilized product.

### BACKGROUND

Honey has been used for thousands of years for wounds. The benefits of honey are that it keeps the wound moist and is antimicrobial. Manuka honey that has been sterilized has been approved by the FDA to be used for a variety of wounds. Honey generally does not contain bacteria due to low pH, low water activity level, and natural bactericidal products. Honey is also used to inhibit stomach ulcers. The problem with honey is that is can harbor bacterial, fungal, or yeast spores. *C. botulinum* is a spore forming bacteria that can create toxins that can cause death and disability. *C. difficile* is a spore forming bacteria that can cause gastrointestinal infections. *B. cerius* is a spore forming bacteria that can cause food poisoning, wound infections, and toxins. Destroying these spores may make honey a more effective medication. There are a variety of ways to sterilize honey. It can be pasteurized, but this is costly and changes the honey. Wound care companies use E-beam radiation. The problem with this is the cost, transportation costs, and limited availability of E-beam radiation. I have invented a new way to sterilize wound honey through a combination of thermal inhibition and chemical sterilization. Heating to sub-Pasteurization temperatures can kill spores, but does not sterilize a product because spores are resistant to freezing and heating. The limitation of chemical sterilization is that it may leaves a toxin or metabolite that is toxic to new forming tissues found in a wound. Parecetic acid is a known sterilant that kills bacteria, fungus, yeast, and spores. It degrades into two natural products found in natural foods, acetic acid and hydrogen peroxide. Parecetic acid and hydrogen peroxide have a synergistic sporicidal effect. Performic acid has demonstrated stronger sporicidal effects than parecetic acid. Performic acid degrades into two natural products found in foods, formic acid and hydrogen peroxide. These two natural products have a synergistic antimicrobial effect. Green tea polyphenols inhibit spore germination. One polyphenol in particular is epigallocatechin gallate. Adding green tea polyphenols will further inhibit bacterial spores.

One example of my invention, that does not limit the scope of this patent, is a honey that is processed with a combination of heating to a level sufficient to inhibit bacterial spores and chemical sterilization. The honey is mixed with the following: parecetic acid at 50 to 50,000 parts per million (0.005% -5%), hydrogen peroxide at a concentration of two hundred to two hundred thousand parts per million (.02% -20%), acetic acid at a concentration of 50 to 50,000 parts per million (.005 - 5%), and formic acid at a concentration 50 to 50,000 parts per million (0.005% - 5%). The honey then is heated between one hundred forty-five (145) and two hundred (200) degrees Fahrenheit (80 Celsius - 93 Celcius) and cooled. Green tea with 50% EGCG will be added at a concentration of 50 to 50,000 parts per million (0.005%-5%) and the wound product may have to rest to allow the parecetic acid to degrade prior to use.

Processed foods that may be sterilized with peracetic acid include, but are not limited to pickled foods, condiments, salad dressing, and marinades.

Other examples of foods that may be sterilized using performic acid include, but are not limited to, apple sauce, processed apple products, processed strawberry products, and processed raspberry products.

Medications that may be sterilized by this process, that does not limit the scope of this patent, include a natural honey antiulcer product that inhibits *H. pylori.*

Some cosmetics contain manuka honey for its natural preservative properties. Cosmetics that may be sterilized by this process, that does not limit the scope of this patent, include a cosmetic that contains honey.

By combining parecetic acid and / or performic acid with heating, I have invented a new means to sterilize products that can be used for food, medication, or cosmetics.

### SUMMARY

This invention includes sterilized through a new, inventive means of combination chemical sterilization using parectic acid and / or performic acid with thermal inhibition.

One example of my unique and beneficial invention, that does not limit the scope of this patent, is a honey in which microbes are inhibited by mixing with the following: parecetic acid between 200 and 2,000 parts per million, hydrogen peroxide at a concentration between 30,000 and 50,000 parts per million, acetic acid at a concentration of 20,000 to 50,000 parts per million, and formic acid at a concentration between 200 and 2,000 parts per million. The honey is heated between one hundred sixty-five to two hundred (200) degrees Fahrenheit (93 Celcius) for more than 36 minutes. After adding green tea with 50% EGCG at a concentration of 1,000 and 15,000 parts per million, this wound product may be rested and tested for a parecetic acid levels before being used. Other products that could benefit from this sterilization process include foods, cosmetics and / or antiulcer products.

Foods that that may be sterilized with peracetic acid and / or performic acid include honey, pickled foods, condiments, salad dressing, marinades, processed strawberry products, and processed raspberry products.

A medicine that can be sterilized with peracidic acid and / or performic acid include a natural antiulcer medication containing honey.

Some cosmetics also contain honey that may benefit from this process.

By combining parecetic acid and / or performic acid with heating, I have invented a new means to sterilize products. Green tea polyphenols may be added to prevent germination of any potential residual bacterial spores. These product may be used for a variety of uses including cosmetics, medicine, and food.

### DETAILED DESCRIPTION

This invention is a honey that is thermally inhibited and sterilized by a chemical sterilant or chemical sterilants. The spores in honey are damaged through heating to reduce the spore count in the product and exposed to a sterilant or sterilants that break down into natural products that are naturally found in honey.

Peracetic acid has a synergistic effect with hydrogen peroxide. The byproducts of percetic acid are the natural bactericide hydrogen peroxide and the natural preservative acetic acid. Parecetic acid has an approximate half-life of 31 hours at room temperature in products with a high concentration of organic matter. Table 1 shows the effects of 0.05% peracetic acid, 1% hydrogen peroxide, and a combination of 0.05% and 1% hydrogen peroxide on *B. subtilis* over time.

**Table 1. Logarithmic decrease in Bacillus subtilis spores with treatment over time.**

| Time | 0.05% Peracetic Acid | 1% Hydrogen Peroxide | 0.05% Peracetic Acid and 1% Hydrogen Peroxide |
|---|---|---|---|
| 1 hours | 1 | 0.25 | 1 |
| 2 hours | 1.5 | 0.5 | 2.25 |
| 3 hours | 1.75 | 0.75 | 3 |

Performic acid has demonstrated stronger sporicidal effects. The byproducts of performic acid, formic acid and hydrogen peroxide, have synergistic antimicrobial effects and are found naturally in honey. Performic acid breaks down very rapidly with a half-life of hours. The synergistic effects of formic acid with hydrogen peroxide is demonstrated in table 2.

**Table 2. The Minimal Bactericidal Concentration of formic acid and acetic acid in combination with hydrogen peroxide in percentage.**

| Acid | Bacteria | Acid/H2O2 | Acid Alone | H2O2 Alone |
|---|---|---|---|---|
| Formic | *Enterococcus hirae* | 0.312% / 0.78% | 5% | 12.5% |
| Formic | *Staphylococcus aureus* | 0.156% / 0.39% | 1.25% | 6.25% |
| Formic | *Listeria monocytogenes* | 0.039% / 0.39% | 0.156% | 3.12% |
| Formic | *Salmonella sp.* | 0.039% / 0.78% | 0.156% | 3.12% |
| Formic | *Escherichia coli* | 0.078% / 0.19% | 0.312% | 1.56% |
| Formic | *Pseudomonas aeruginosa* | 0.0015% / 0.195% | 0.025% | 1.56% |
| Acetic | *Enterococcus hirae* | 2.5% / 3.12% | 10% | 12.5% |
| Acetic | *Staphylococcus aureus* | 1.25% / 1.56% | 5% | 6.25% |
| Acetic | *Listeria monocytogenes* | 1.25% / 1.56% | 5% | 3.12% |
| Acetic | *Escherichia coli* | 0.625 / 0.31 | 2.5% | 1.56% |
| Acetic | *Pseudomonas aeruginosa* | 0.156 / 3.12 | 0.625% | 1.56% |

Green tea polyphenols have demonstrated the ability to inhibit bacterial spores. Table 3 demonstrates the ability of EGCG and green tea polyphenols to inhibit *Bacillus subtilis* spores.

**Table 3. Percentage of inhibition of Bacillus subtilis spores.**

| Percentage | EGCG | Green Tea Polyphenols |
|---|---|---|
| 1% | 98.53% | 94.2% |
| 5% | 98.77% | 100% |

One example of my unique and beneficial invention, that does not limit the scope of this patent, is a honey that is mixed with the following: parecetic acid at 10,000 parts per million (1%), hydrogen peroxide at a concentration of 14,667 parts per million (1.5 %), acetic acid at a concentration of 10,667 parts per million (1.1%), and formic acid at a concentration of 1,500 parts per million (0.15%). After resting 48 hours, the honey is sterilized by heating to two hundred (200) degrees Fahrenheit (93 Celcius) for 40 minutes. Green tea with 50% EGCG at a concentration of 20,000 parts per million (2%) will be mixed with the honey before resting for 2 weeks and then tested for spore and peracetic acid levels. This product is designed to be used for wounds.

Other products included in this patent but not limiting the scope of this patent, includes foods, cosmetics, and medications. Examples of these products include, but are not limited to: table honey, pickled foods, condiments, salad dressings, marinades, processed strawberry products, processed raspberry products, natural antiulcer medication, and cosmetics that contain honey.

Please note that this patent is not limited by timeframe or order of processing. For example, the product may be heated before or after the chemical additives are added. The product may be rested for a shorter time or longer time in order to improve time to market or improve efficacy of the sterilization process or to give more time for the breakdown of the sterilization chemicals.

## Claims

1. A food, cosmetic, or medication mixed or percolated with at least one of a combination of formic acid and hydrogen peroxide, a combination of acetic acid and hydrogen peroxide, peracetic acid, and performic acid, in sufficient quantity to inhibit or inactivate spores.

2. The above mentioned product in claim #1 that kills or inactivates spores after heating to less than 121 degrees Celsius.

3. The above mentioned product in claim#1 that kills or inactivates bacterial spores after sitting for more than 48 hours.

4. The above mentioned product in claim #1 that is combined with an antimicrobial or antimicrobials that inhibit or inactivates spores.

5. The process of mixing or percolating the ingredients in claim #1

6. The process of heating honey or a product containing honey and using a chemical to kill or inactivate spores.

7. The above mentioned product in claim #6 that kills or inactivates bacterial spores after at least one of heating to less than 121 degrees Celsius.

8. The above mentioned product in claim#6 that kills or inactivates bacterial spores with the addition of acid or acids.

9. The above mentioned product in claim#6 that kills or inactivates bacterial spores after sitting for 48 hours.

10. The above mentioned product in claim #6 that is combined with an antimicrobial or antimicrobials that inhibit or inactivates spores.

11. The process of mixing the ingredients in claim #6.

12. Honey sterilized with at least one chemical.

13. The above mentioned product in claim #12 that kills or inactivates bacterial spores after heating to less than 121 degrees Celsius.

14. The process of mixing the ingredients in claim #12.
